# EUROPEAN PATENT APPLICATION

(11) **EP 3 192 440 A1**
(43) Date of publication of application: **19.07.2017**
(21) Application number: 15839369.4
(22) Date of filing: 10.09.2015
(51) Int. Cl.: A61B 5/04, A61B 5/042

(54) **CATHETER AND METHOD FOR DETECTING ELECTRICAL ACTIVITY IN AN ORGAN**

(30) Priority: 12.09.2014 ES 201431315
(71) Applicant: Universidad Politécnica de Valencia, 46022 Valencia (ES); Fundación para la Investigación Biomédica del Hospital Gregorio Marañon, 28007 Madrid (ES); The Regents of The University of Michigan, Ann Arbor, Michigan 48109 (US)
(72) Inventor: RODRIGO BORT, Miguel, E-46022 Valencia (ES); MARTINEZ CLIMENT, Batiste Andreu, E-28007 Madrid (ES); GUILLEM SANCHEZ, Maria de la Salud, E-46022 Valencia (ES); ATIENZA FERNANDEZ, Felipe, E-28007 Madrid (ES); BERENFELD, Omer, Ann Arbor, Michigan 48109-2590 (US)
(74) Representative: Maslanka Kubik, Dorota Irena
(86) International application number: PCT/ES2015/070657
(87) International publication number: WO 2016/038237

(57) **Abstract**

The present invention relates to a catheter and method for detecting electrical activity in an organ. The catheter comprises: a proximal end with connection means for connecting to a signal processing system and a distal end for being inserted into a patient's organ, at least 3 arms extending from the distal end, each arm comprising at least one electrode. The catheter further comprises a central electrode at the distal end of the catheter. The method comprises: inserting a multi-electrode catheter into a patient's organ; obtaining and conditioning a positioning signal for positioning the electrodes; obtaining causal information about the current location; processing and summarizing the causal information obtained together with causal information about previous locations; visually presenting a recurrence plot of all the causal information obtained; and then moving the catheter to a new location and repeating the method until obtaining a complete recurrence plot.

## Description

### TECHNICAL FIELD

The present invention generally relates to the field of medicine; it more specifically relates to a catheter and method for detecting electrical activity in a patient's organ, more specifically for detecting electrical activity in the heart, for example in a patient suffering cardiac fibrillation.

### PRIOR ART

Cardiac fibrillation is a type of complex arrhythmia having mechanisms of onset, continuance and interruption that are not completely known. Although it was considered for a long time that fibrillation processes were random phenomena with no coordinated pattern, recent studies have demonstrated that in some cases there may be a hierarchical pattern defining the fibrillation process. Therefore, the electrical isolation of the dominant area can be an effective treatment for heart arrhythmias of this type (see M. Rodrigo Bort et al., Mapa de relación causal: una nueva metodología para la identificación de patrones fibrilatorios jerárquicos, XXIX Congreso Anual de la Sociedad Española de Ingenieria Biomédica (29th Annual Congress of the Spanish Biomedical Engineering Society).

The prior art discloses methods and systems for detecting the optimal area for treating the arrhythmia. These methods are generally based on detecting and presenting to a user the dominant frequency and heart signal fractionation. However, these dominant frequency-based methods require the user (the physician) to move the catheter throughout the entire atrium to record a signal in all of said atrium for the purpose of detecting the optimal location for ablation treatment. This requires a long time, which increases patient risks in an invasive treatment.

These systems further require the physician performing the procedure to have undergone highly specialized training and to be extremely knowledgeable in order to interpret the parameters that are obtained.

For example, patent document WO2012092016 discloses a system for diagnosing arrhythmias and directing catheter therapies which allows measuring, classifying, analyzing and mapping spatial electrophysiological patterns inside a body. The system may further guide arrhythmia therapy and update maps as treatment is delivered. The system may use a medical device having a high density of sensors with a known spatial configuration for collecting electrophysiological data and positioning data. Further, the system may also use an electronic control system for computing and providing the user with a variety of metrics, derivative metrics, high definition maps, composite maps, and general visual aids for association with a geometrical anatomical model shown on a display device.

However, although the method disclosed in patent document WO2012092016 may help to direct arrhythmia catheter treatment to an optimal location, the possibility of improvement continues to exist with respect to the precision of the data provided to the user.

Therefore, there is still a need in the art for an alternative method for detecting electrical activity in an organ of the human body (for example, electrical activity in the heart) that provides visual aids and precise data to a user, for example a physician, performing for example an arrhythmia catheter ablation treatment.

In particular, several types of catheters for performing heart arrhythmia treatments are also known in the art. For example, patent document US 6,961,602 discloses a catheter for mapping electrical activity in the heart. The catheter comprises a plurality of arms which can each obtain electrical, mechanical and location data. The catheter comprises an elongated catheter body having proximal and distal ends and at least one lumen extending longitudinally therethrough. A mapping assembly having at least two arms is mounted at the distal end of the catheter body, each one having a proximal end attached to the distal end of the catheter body and a free distal end. Each arm comprises at least one location sensor and at least one tip electrode and a ring electrode. In use, at least one electrode of each arm is placed in contact with heart tissue for mapping the electrical activity of the heart. Location sensors are used for determining the location of each point where electrical activity is monitored.

However, the mapping information obtained with an electrode of this type is not optimal because a large number of errors are introduced in the measurements which originate from, for example, relative locations of the electrodes of the arms with respect to one another.

Therefore, there continues to be a need in the art for an alternative catheter for detecting the electrical activity in an organ of the human body (for example, electrical activity in the heart) that reduces the detection errors occurring when measuring various heart activity parameters.

### DISCLOSURE OF THE INVENTION

To solve the problems of the prior art, the present invention discloses a catheter and a method for detecting electrical activity in an organ.

According to a first aspect, the present invention discloses a catheter for detecting electrical activity in an organ comprising a proximal end with connection means for connecting to a signal processing system and a distal end intended to be inserted into a patient's heart. The catheter further comprises at least 3 arms extending from the distal end, each arm comprising at least one electrode. The catheter further comprises a central electrode at the distal end from where each of the arms of the catheter emerges.

According to the catheter of the present invention, the distance between the electrodes of each of the arms and the central electrode is known. The central electrode is therefore used as a reference electrode for the measurements taken by the electrodes of the arms. As a result of the catheter of the invention, it is therefore possible to take causal measurements in various directions, i.e., between the central electrode and each of the electrodes of the arms. Given that the reference electrode is the same for all the measurements and the distance between the reference electrode and the electrodes of the arms is known, errors introduced by the measurement are substantially reduced. Furthermore, given that the catheter comprises at least three arms with electrodes, causal measurements of the electrical activity in at least three directions can be obtained, so precision increases and mapping the electrical activity in the organ is made substantially easier.

According to a second aspect, the present invention discloses a method for detecting electrical activity in an organ. The method comprises the steps of:
a) inserting a multi-electrode catheter into a patient's organ;
b) obtaining and conditioning a positioning signal for positioning electrodes of the catheter inside the organ;
c) obtaining causal information about the location where the catheter can be found;
d) processing and summarizing the causal information obtained together with causal information obtained at previous locations of the catheter;
e) visually presenting to the user a recurrence plot of all the causal information obtained up to the present, which summarizes the electrical activity of the organ in a collective manner; and
f) moving the catheter to a new location and repeating the method from step b) above until a complete and satisfactory recurrence plot of the patient's organ is obtained.

The method of the invention therefore provides the user (i.e., the physician who will be performing, for example, a heart arrhythmia ablation treatment) with precise and detailed information about the electrical activity in the organ in the form of a causal activity map, whereby the user can direct the catheter, for example, directly to the optimal area where arrhythmia treatment must be performed.

Given that the causal information obtained by the catheter is processed and summarized together with the information obtained in other previous locations in the organ, the recurrence plot presented to the user will be updated as the catheter moves around in the organ, and therefore precision of the information presented therein is completed and improved.

### BRIEF DESCRIPTION OF THE DRAWINGS

The present invention will be better understood in reference to the following drawings illustrating preferred embodiments of the invention provided by way of example and which must not be interpreted as being limiting of the invention in any way.
Figures 1A, 1B and 1C show three embodiments of the catheter according to the present invention.
Figures 2A, 2B and 2C show three other embodiments of the catheter according to the present invention.
Figure 3 shows a flow chart of a method according to the preferred embodiment of the present invention.
Figure 4 shows a recurrence plot of an atrium obtained by means of the method according to the preferred embodiment of the present invention.

### DETAILED DISCLOSURE OF THE PREFERRED EMBODIMENTS

A detailed description of preferred embodiments of the catheter and the method of the present invention, specifically applied to detecting electrical activity in the heart for the purpose of determining the optimal location of treatment, for example, of a heart arrhythmia, is provided below. The person skilled in the art will nevertheless understand that this application is not at all limiting of the present invention, and that the catheter and the method disclosed herein can be applied to detecting electrical activity in other organs of a patient's body, whether the patient is a human being or other animal.

As mentioned above, the catheter according to the present invention comprises a proximal end and a distal end. The proximal end has connection means for connecting to a signal processing system. These connection means can be any means that are known and commonly used in the art, such as connection means for connecting to a computer or any signal processing computer system. The obtained signals are preferably processed by means of a method according to the present invention, as will be described herein below.

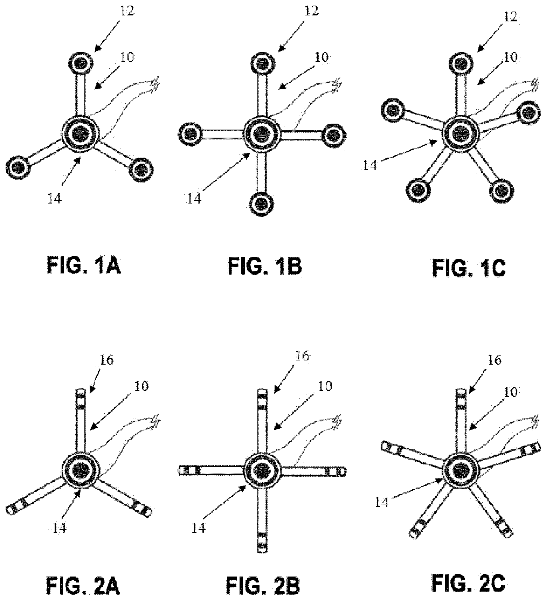

The distal end of the catheter comprises at least 3 arms extending from same, each of which comprises at least one measurement electrode. Figures 1A to 1C show three preferred embodiments of the distal end of the catheter of the present invention, having 3, 4 and 5 arms (10), respectively. In this case, as can be seen, each arm (10) has a Laplacian electrode (12) at the end thereof. The catheter also comprises a central electrode (14) at its distal end from where each of the arms (10) of the catheter emerges.

According to these preferred embodiments, regardless of the number of arms (10), the electrodes (12) of the arms (10) are equidistant with respect to the central electrode (14) and with respect to the electrodes (12) of the contiguous arms (10) thereof.

Figures 2A to 2C show three other embodiments of the catheter of the present invention. The embodiments of Figures 2A to 2C are similar to those of Figures 1A to 1C, respectively, the difference being that the Laplacian electrodes (12) of the arms are replaced with bipolar electrodes (16). Although the central electrode (14) in Figures 2A to 2C is still a Laplacian electrode, the person skilled in the art will understand that embodiments according to the present invention in which said central electrode is replaced with one of bipolar type could also be conceived.

However, according to the present invention the use of Laplacian electrodes both in the arms and in the center of the distal end of the catheter is generally preferred, because they can more effectively eliminate the far field component of the recorded electrical signal of the heart and therefore reduce the error in the measurement. Correct analysis of local activity of the location of the cardiac wall where the electrodes of the catheter are located is therefore allowed at all times without the influence of the electrical activity of other parts of the heart muscle.

According to an additional embodiment of the present invention not shown in the drawings, the catheter further comprises a set of three coils located in each arm close to the corresponding electrode thereof. The presence of this set of coils may make spatial location of the electrodes inside a patient's heart easier, as will be described herein below.

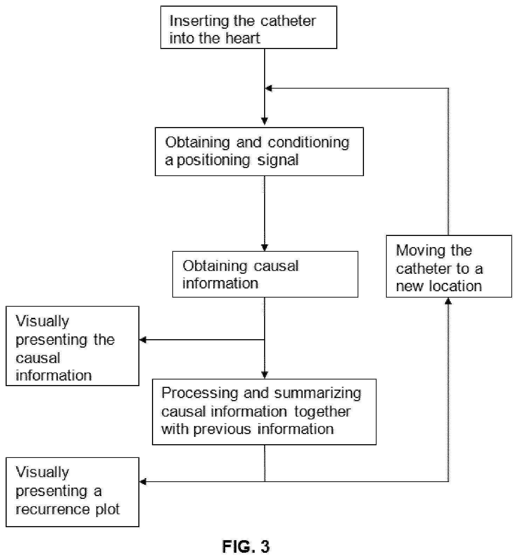

Referring to Figure 3, a method for detecting electrical activity of the heart according to a preferred embodiment of the present invention is described. The method comprises the steps of:
a) inserting a multi-electrode catheter, for example a catheter according to the present invention, into a patient's heart;
b) obtaining and conditioning a positioning signal for positioning electrodes of the catheter inside the heart;
c) obtaining causal information about the location where the catheter can be found;
   c2) visually presenting to the user the causal information obtained up to the present
d) processing and summarizing the causal information obtained together with causal information obtained at previous locations of the catheter;
e) visually presenting to the user a recurrence plot of all the causal information obtained up to the present, which summarizes the electrical activity of the heart in a collective manner; and
f) moving the catheter to a new location and repeating the method from step b) above until a complete and satisfactory recurrence plot of the patient's heart is obtained.

The method according to this preferred embodiment of the present invention can be applied, for example, for detecting a fibrillation source area that must be treated by means of ablation.

Step a) of inserting the catheter into a patient's heart is similar to the one performed with catheters known in the prior art and therefore does not require being further described.

After step b), the method of the invention is an iterative method. In step b), the electrodes of the catheter are located spatially, i.e., the exact location of each of the electrodes inside the patient's heart is determined. This step can be done in several ways.

For example, a method for obtaining positioning signals for positioning the electrodes in the patient's heart consists of placing 3 electrical references on the patient, in the form of electrodes on the skin, and circulating an electric current between each of them and each of the electrodes for obtaining a signal of the catheter. In this manner, by measuring the impedance between each of the electrodes and each of the references, the three-dimensional coordinates of the electrodes with respect to the position of the references can be obtained. The signal circulating through the electrode for location purposes must not overlap with the frequency spectrum of the signal of interest that is being measured because in that case the intracardiac signal obtained would not be correct.

Another way to obtain this information concerning the location of the electrodes of the catheter consists of using a catheter comprising, close to each electrode of each arm, a set of three coils such as mentioned herein above. These coils allow measuring strength of the electromagnetic field created by other coils that are placed on the patient's back. These other larger-sized coils are placed either adhered to the patient's back or on the operating table and they create a magnetic field which induces an electric current in the smaller coils placed next to each electrode of the catheter. The measurement of this electric current induced in the coils placed in the catheter allows obtaining the position thereof with respect to the larger coils.

As a person skilled in the art will comprehend, conditioning of the obtained location signal is preferably performed (by means of any of the methods mentioned above or by another method known in the art), so temporary filters that allow removing electronic noise, as well as any other type of signal processing which increases signal quality, for example, are included.

Once the location of each of the electrodes of the arms of the catheter is thereby determined, and given that the distance between each electrode of each arm and the central electrode is known (preferably, the distance between each electrode of the arms and the central electrode is constant), the position of all the electrodes inside the patient's heart is fully determined.

In the event of using a catheter having more than three arms, a set of three coils can be included in each arm, or a set of three coils can be included in only three of the arms of the catheter. Indeed, if the relative position of the electrodes in the arms of the catheter and the central electrode is constant, upon determining the spatial location of three of the electrodes thereof the location of the remaining electrodes of the catheter is deduced immediately.

Step c) obtains causal information about the location where the catheter can be found at that time. To that end, the physician places the distal end of the catheter with the recording points on a specific point on the surface of the heart (for example, of the atrium), and after determining the location of the electrodes (step b) mentioned above), the myocardial signal is recorded for a brief time period (for example between 1 and 10 seconds). This signal is recorded and processed, first obtaining the causal information of that specific area of the atrium where the distal end of the catheter is located. This causal information preferably includes the dominant propagation direction, the fibrillation activity organization index and other parameters derived from these measurements.

Step c) consists of obtaining and processing causal information about the location where the catheter can be found at that time. To take said causality measurement the signal is segmented into temporal windows on which the analysis will be performed. The final result is the combination of the result obtained in each of these temporal windows. There are several ways to segment the signal. According to one embodiment of the invention, segmentation is performed in intervals equal to the inverse of the dominant cardiac activation frequency. To that end, the dominant frequency of each of the recorded heart signals must be calculated using the method known in the art as Welch's periodogram, for example. Once this dominant frequency value is known, the signals are cut up into overlapping segments with a temporal length equal to the inverse of the highest dominant frequency among all the heart signals (the overlap interval is a factor that the user must introduce depending on signal duration and on expected robustness). Once the signal is segmented, the causal influence between each pair of signals in each temporal window is calculated, i.e., between the heart signals of the center of the catheter and the heart signals of the electrodes of the arms of the catheter. This influence will be proportional to the value of the error variance produced in the prediction upon applying an autoregressive model derived directly from the definition of Granger causality. Parameters directly dependent on the distance between electrodes are included in this autoregressive model, so it is preferable to use a catheter in which the electrodes of each of the arms are always equidistant to one another and with respect to the central electrode. The calculated values of causal influence between the heart signals obtained by the various electrodes will be used to obtain dominant propagation direction and fibrillation activity organization index parameters.

According to the preferred embodiment of the present invention, step c2) is performed next, wherein the causal information obtained up to the present, i.e., the propagation direction and organization index parameters calculated in the previous step for this location in the atrium, is visually presented to the user. However, the person skilled in the art will understand that this step is not mandatory for the proper working of the method, and therefore in alternative embodiments of the invention the method goes directly from step c) to step d) described herein below. To perform step c2), the position of the catheter inside the atrium is used, displaying on a virtual map of the atrium the parameters mentioned in the form of a set of arrows indicating the direction, or a single arrow, together with the organization value represented, for example, with a color map or directly with the value thereof on the display. Additionally, other parameters of the heart signal not derived from the causal analysis, but rather parameters characteristic of the area of the atrium where the catheter can be found, such as, for example, the heart signal itself, the amplitude of the heart signals obtained, the dominant frequency thereof, the degree of fractionation of said signals, the relative position of the catheter with respect to the atrium, etc., can also be shown in this step.

Next in step d), the causal information obtained together with causal information obtained at previous locations of the catheter on the wall of the atrium is processed and summarized. To that end, a three-dimensional mesh model detailing the morphology of the wall of the atrium, defined by its nodes or points in space is used. This model is obtained from the information about the spatial position of the catheter throughout the intervention. Based on the information about the dominant propagation direction obtained from the causal analysis for each location of the catheter, a dominant propagation direction value is obtained for each node of the atrial mesh. A Markov chain-based model is built with this information, in which each spatial node of the atrial mesh is the equivalent to a state of the Markov chain, and the probabilities associated with changes in state are obtained from the spatial information of the model together with the dominant propagation directions calculated for each node. The final probability distribution among the different nodes is then calculated for the stationary phase of the Markov chain, defining a uniform distribution among the different nodes as an initial probability. This final probability distribution among nodes is called a recurrence plot and it has a value for each node of the mesh of the surface of the atrium between 0 and 1.

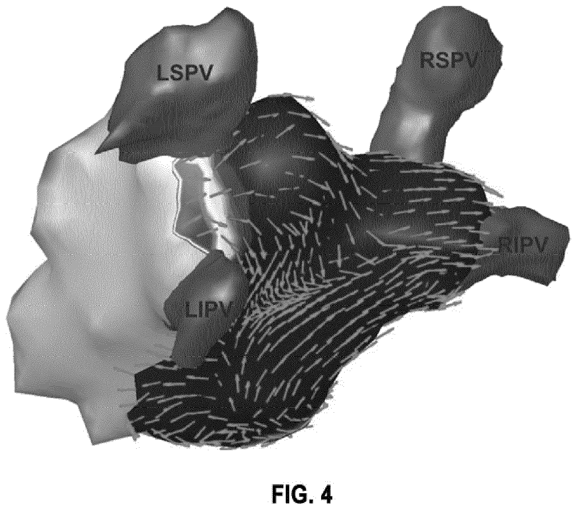

In step e), a recurrence plot of all the causal information obtained up to the present, which summarizes the electrical activity of the heart in a collective manner, is visually presented to the user. This map displays the virtual model of the atrium with the dominant propagation direction of each node shown with an arrow, and a color code can also be used on the very surface of the atrium to show the values of the recurrence plot (see Figure 4). Furthermore, like in step c2) described herein above, other information such as, for example, the recorded heart signal, dominant frequency, etc., can also be added to the display.

Finally, step f) consists of moving the distal end of the catheter to a new location and then repeating the method from step b) above until a complete and satisfactory recurrence plot of the patient's heart is obtained. According to the preferred embodiment of the method of the present invention, in this step the user is shown a typical browsing display of an electroanatomic recording system, in which the virtual model of the atrium together with the relative position of the catheter are shown.

As can be appreciated by the person skilled in the art, the present method is a huge leap forward with respect to the prior art methods which are generally based on the dominant frequency and on heart signal fractionation, as discussed herein above. Indeed, on one hand the present method allows obtaining and visually presenting to the user the dominant propagation direction, i.e., the existing propagation pattern during atrial fibrillation in an area of the wall of the atrium, which is highly relevant information in arrhythmia treatment. Knowledge about this information allows the physician to effectively guide ablation catheter correctly and rapidly to an optimal area of application, and therefore allows preventing moving all over the entire surface of the atrium to record a signal from all of said surface, as must be done with prior art methods.

On the other hand, the method of the present invention allows a more intuitive analysis of the propagation pattern by the physician, which can facilitate and reduce the duration of the intervention, and requires a lower level of attending physician training and know-how than in prior art methods.

Furthermore, as discussed above, the method of the present invention summarizes the activity recorded in a single map (recurrence plot). The attending physician can easily and rapidly comprehend this recurrence plot, which facilitates treatment and additionally reduces intervention time. In a single view, this map shows both the propagation patterns detected on the surface of the atrium and hierarchically dominant areas, so atrial activity during fibrillation as well as the areas of interest for ablation can be observed in a simple manner.

The catheter and method of the present invention described above can be applied, for example, in electrophysiology laboratories as a diagnostic and therapeutic tool in patients. As discussed above, one of the possible applications of the catheter and method of the present invention (although there can also be other similar applications) is the detection of regions of the heart causing the onset and/or continuance of the arrhythmic process and therefore susceptible to being cauterized for the purpose of putting an end to the arrhythmia. Included among those pathologies that can be detected and/or treated by means of the catheter and method of the present invention are, for example, atrial fibrillation, atrial flutter, focal atrial and/or ventricular tachycardias, and ventricular tachycardias.

Although the present invention has been described referring to preferred embodiments thereof, modifications and variations that are evident to the person skilled in the art can be applied without departing from the scope of the present invention. For example, although a catheter and a method applied to the detection of electrical activity in the heart for determining, for example, an optimal heart arrhythmia treatment area have been described, the person skilled in the art understands that the present invention can also be applied to the detection of electrical activity in other organs, such as in muscles or in nerve tissues, for example.

Likewise, according to the preferred embodiments of the invention, it has been described that the electrodes in the arms of the catheter are equidistant to one another and with respect to the central electrode. However, in alternative embodiments of the present invention a catheter can be designed in which the electrodes of the arms are only equidistant to one another, are only equidistant with respect to the central electrode or are not equidistant with respect to any other electrode of the catheter. However, in the case in which the electrodes of the arms are not equidistant with respect to the central electrode, according to the present invention said distance between the electrodes of each arm and the central electrode must be known.

In addition, catheters comprising three, four and five arms with electrodes therein have been described. However, the person skilled in the art will understand that catheters with a larger number of arms can be designed without departing from the scope of the present invention. Likewise, each of said arms can comprise more than one electrode therein.

## Claims

1. A catheter for detecting electrical activity in an organ, comprising a proximal end with connection means for connecting to a signal processing system and a distal end intended for being inserted into a patient's organ, the catheter further comprising at least 3 arms (10) extending from the distal end, each arm comprising (10) at least one electrode (12, 16), **characterized in that** it further comprises a central electrode (14) at the distal end of the catheter, from where each of the arms (10) of the catheter emerges.

2. The catheter according to the preceding claim, **characterized in that** the central electrode (14) is a Laplacian electrode.

3. The catheter according to any of the preceding claims, **characterized in that** it comprises between 3 and 5 arms (10), each arm (10) comprising at least one electrode (12, 16).

4. The catheter according to any of the preceding claims, **characterized in that** the electrodes (12) in the arms (10) of the catheter are Laplacian electrodes.

5. The catheter according to any of the preceding claims, **characterized in that** the electrodes (12, 16) of each arm (10) of the catheter are equidistant with respect to the central electrode (14).

6. The catheter according to any of the preceding claims, **characterized in that** the electrodes (12, 16) of each arm (10) of the catheter are equidistant with respect to the electrodes (12, 16) of the contiguous arms (10) thereof.

7. The catheter according to any of the preceding claims, **characterized in that** it further comprises a set of three coils located in at least three arms (10), close to the corresponding electrode (12, 16) thereof.

8. The catheter according to any of the preceding claims, **characterized in that** it is intended for detecting electrical activity in the heart.

9. A method for detecting electrical activity in an organ, comprising the steps of:
a) inserting a multi-electrode catheter into a patient's organ;
b) obtaining and conditioning a positioning signal for positioning electrodes of the catheter inside the organ;
c) obtaining causal information about the location where the catheter can be found;
d) processing and summarizing the causal information obtained together with causal information obtained at previous locations of the catheter;
e) visually presenting to the user a recurrence plot of all the causal information obtained up to the present, which summarizes the electrical activity of the organ in a collective manner; and
f) moving the catheter to a new location and repeating the method from step b) above until a complete and satisfactory recurrence plot of the patient's organ is obtained.

10. The method according to claim 9, **characterized in that** it comprises an additional step c2) of visually presenting to the user the causal information obtained up to the present.

11. The method according to any of claims 9 and 10, **characterized in that** the causal information obtained and presented to the user includes the dominant electrical propagation direction.

12. The method according to any of claims 9 to 11, **characterized in that** the causal information obtained and presented to the user includes the fibrillation activity organization index.

13. The method according to any of claims 9 to 12, **characterized in that** the method is applied for detecting a fibrillation source area that must be treated by means of ablation.

14. The method according to any of claims 9 to 13, **characterized in that** step b) of obtaining and conditioning a positioning signal for positioning the electrodes comprises placing three reference electrodes on the patient and circulating an electric current between each of them and each of the electrodes of the arms of the catheter, measuring impedance between each of the electrodes of the arms of the catheter and each reference electrode and obtaining the three-dimensional coordinates of the electrodes of the arms of the catheter with respect to the position of the reference electrodes.

15. The method according to any of claims 9 to 13, **characterized in that** step b) of obtaining and conditioning a positioning signal for positioning the electrodes comprises placing large coils on the patient's back, measuring an electric current induced by the large coils in sets of three coils included next to each electrode of the catheter and determining the position of the sets of three coils with respect to the large coils.

16. The method according to any of claims 9 to 15, **characterized in that** it comprises using a catheter according to any of claims 1 to 8.

17. The method according to any of claims 9 to 16, **characterized in that** the organ the electrical activity of which is detected is the heart.
